# EUROPÄISCHE PATENTSCHRIFT

(11) **EP 2 601 171 B1**
(45) Veröffentlichungstag und Bekanntmachung des Hinweises auf die Patenterteilung: **03.09.2014**
(21) Anmeldenummer: 11743479.5
(22) Anmeldetag: 20.07.2011
(51) Int. Cl.: C07C 219/06, C07C 235/74

(54) **BETA-KETOCARBONYLQUATVERBINDUNGEN UND VERFAHREN ZU DEREN HERSTELLUNG**
BETA-KETOCARBONYLQUAT COMPOUNDS AND PROCESS FOR THE PREPARATION THEREOF
COMPOSÉS QUATERNAIRES DE BÊTA-CÉTOCARBONYLE ET PROCÉDÉS POUR LEUR PRÉPARATION

(30) Priorität: 04.08.2010 DE 102010038887
(43) Veröffentlichungstag der Anmeldung: 12.06.2013
(73) Patentinhaber: Wacker Chemie AG, 81737 München (DE)
(72) Erfinder: HERZIG, Christian, 83329 Waging (DE)
(74) Vertreter: Deffner-Lehner, Maria
(86) Internationale Anmeldenummer: PCT/EP2011/062465
(87) Internationale Veröffentlichungsnummer: WO 2012/016837

(56) Entgegenhaltungen:
- US-A1- 2009 247 629
- BUCHI J ET AL: "Synthesis and action of several curare-like diammonium derivatives with ester, ketone, and alcohol functions", ARZNEIMITTEL FORSCHUNG. DRUG RESEARCH, ECV EDITIO CANTOR VERLAG, AULENDORF, DE, Bd. 10, Nr. 9, 1. Januar 1960 (1960-01-01) , Seiten 699-709, XP008143988, ISSN: 0004-4172

## Beschreibung

Gegenstand der Erfindung sind Quatverbindungen, d. h. Verbindungen, die mindestens eine quartäre Ammoniumgruppe enthalten, und Verfahren zu deren Herstellung.

EP 1 259 672 B1 beschreibt die Verwendung von Alkyl-Keten-Dimer-Dispersionen zur Antiknitterausrüstung von Textilien. Beschrieben sind auch kationische Emulgatoren, die aus Alkyl-Keten-Dimer und Polyethylenimin in einer Teilumsetzung hergestellt werden. Bis auf diese Teilumsetzungsprodukte, in denen einige Amingruppen des Polyethylenimin amidiert werden , bleibt das dispergierte Alkyl-Keten-Dimer intakt und wird als solches auf die Faser gebracht. Eine Quaternierung des teilumgesetzten Polyethylenimin-Emulgators ist nicht beschrieben und wäre auch wegen des hohen Prozentsatzes von prim. und sec. Aminogruppen kaum wirtschaftlich durchzuführen.

Alkyl-Keten-Dimer-Dispersionen als Textilhilfsmittel sind in folgenden Dokumenten beschrieben:
CH 388246 beschreibt die Verwendung von Formulierungen, die den Ketenweichmacher "Aquapel" 380 (Hercules Powder Co.) zur Imprägnierung und Hydrophobierung von Textilien enthalten. Auch in US 5,028,236 werden Alkyl-Keten-Dimere zur Hydrophobierung von Wolle und synthetischen Polyamidfasern eingesetzt. Mit demselben Ziel werden in WO 96/26318 Alkyl-Keten-Dimer-Dispersionen als Leimungsmittel für Papier, also zur kontrollierten Hydrophobierung, verwendet.

WO 2009/121751 beschreibt die Umsetzung von Aminosiloxanen mit Alkyl-Keten-Dimeren zur Herstellung von Wachsen mit sehr hohem Polydimethylsiloxan-Gehalt. Quaternäre Verbindungen können damit nicht hergestellt werden.

US 2009/247629 A1 beschreibt die Herstellung polymerer organischer Quatverbindungen, die sich durch Veresterung von Dimersäure mit Dimethylaminoalkanol und nachfolgendem Umsatz mit Polyetherepichlorhydrinen herstellen lassen. Die Synthesefolge ist relativ aufwändig und bei hoher Temperatur (200°C) über viele Stunden (3-8 h) auszuführen, also wenig attraktiv.

Es bestand die Aufgabe Quatverbindungen, d. h. Verbindungen, die mindestens eine quartäre Ammoniumgruppe enthalten, bereitzustellen, die in einem einfachen Verfahren schnell und nahezu quantitativ hergestellt werden können und die einen längerkettigen Kohlenwasserstoffrest, vorzugsweise Fettsäurerest, aufweisen.
Die Aufgabe wird durch die Erfindung gelöst.

Gegenstand der Erfindung sind β-Ketocarbonylquatverbindungen, die eine oder mehrere β-Ketocarbonylgruppen der allgemeinen Formel

R-CH₂-(C=O)-CHR-(C=O)- (I)

und
eine oder mehrere quartäre Ammoniumgruppen enthalten, wobei
- R: gleich oder verschieden sein kann und einen aliphatischen Kohlenwasserstoffrest mit 6 bis 28 C-Atomen, vorzugsweise 10 bis 26 C-Atomen, bevorzugt 12 bis 20 C-Atomen, bedeutet,
mit der Maßgabe, dass die β-Ketocarbonylgruppe der Formel (I) an einen Rest Y gebunden ist, wobei
- Y: einen zweiwertigen Rest der Formel -O-, -NH-, -NR¹-, bevorzugt -NH-, -NR¹-, oder einen dreiwertigen Rest der Formel =N- bedeutet, und
- R¹: einen einwertigen Kohlenwasserstoffrest mit 1 bis 30 C-Atomen, vorzugsweise 1 bis 18 C-Atomen, bedeutet.

Quartäre Ammoniumgruppen (quaternary ammonium groups) sind

Derivate der Ammoniumgruppe, bei denen alle vier Wasserstoffatome durch vier N-C-gebundene (ggf. substituierte) Kohlenwasserstoffgruppen, wie Alkylgruppen, ersetzt sind.

Die erfindungsgemäßen β-Ketocarbonylquatverbindungen enthalten vorzugsweise ein oder zwei quartäre N-Atome, bevorzugt ein quartäres N-Atom.
Sie haben ein Molekulargewicht (Mₙ) von vorzugsweise mindestens 300 Dalton, bevorzugt 500 bis 2000 Dalton. Die bevorzugte Ladungsdichte liegt bei 0,5 bis 2,0 mEquiv. N⁺/g.

Bevorzugt als β-Ketocarbonylquatverbindungen sind solche der allgemeinen Formel

[R³R⁴R⁵N⁽⁺⁾-R²-]ₐY-Z X⁽⁻⁾ (II),

wobei
- a: 1 oder 2 ist, mit der Maßgabe, dass wenn a=1, Y ein zweiwertiger Rest ist und wenn a=2, Y ein dreiwertiger Rest ist,
- Y: einen zweiwertigen Rest der Formel -O- , -NH- , -NR¹- , oder einen dreiwertigen Rest der Formel =N- bedeutet,
- X⁽⁻⁾: Gegenion zu der positiven Ladung am quartären Stickstoffatoms ist,
- Z: eine β-Ketocarbonylgruppe der Formel R-CH₂-(C=O)-CHR-(C=O)- (I) bedeutet,
- R: die oben dafür angegebene Bedeutung hat,
- R¹: einen einwertigen Kohlenwasserstoffrest mit 1 bis 30 C-Atomen, vorzugsweise 1 bis 18 C-Atomen, bedeutet,
- R²: einen zweiwertigen C₁-C₁₈-Kohlenwasserstoffrest, der ein oder mehrere separate Sauerstoffatome enthalten kann, bedeutet,
- R³: gleich oder verschieden ist und einen einwertigen Kohlenwasserstoffrest mit 1 bis 30 C-Atomen, vorzugsweise 1 bis 18 C-Atomen, bedeutet,
- R⁴ gleich R³: ist oder einen Rest der Formel -R¹-Y-Z oder -R¹-N⁽⁺⁾R³₂R⁵ X⁽⁻⁾ bedeutet, oder R³ und R⁹ zusammen oder zwei Reste R³ zusammen einen zweiwertigen C₃-C₁₂-Kohlenwasserstoffrest, der gegebenenfalls ein O-Atom oder N-Atom enthalten kann, bedeuten und
- R⁵: einen einwertigen Kohlenwasserstoffrest mit 1 bis 18 C-Atomen, vorzugsweise 1 bis 12 C-Atomen, bedeutet.

Gegenstand der Erfindung ist weiterhin ein Verfahren zur Herstellung der β-Ketocarbonylquatverbindungen,
indem in einem ersten Schritt
Alkyl-Keten-Dimere (1) der allgemeinen Formel 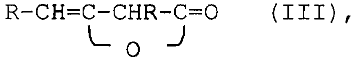 wobei R die oben dafür angegebene Bedeutung hat, mit Aminoverbindungen (2), die mindestens eine tertiäre Aminogruppe und mindestens eine protische Gruppe ausgewählt aus der Gruppe der Formeln -OH, -NH₂, -NHR¹ und -NH-, vorzugsweise -NH₂, -NHR¹ und -NH-, enthalten, wobei R¹ die oben dafür angegebene Bedeutung hat,
umgesetzt werden, wobei tertiäre Aminogruppen aufweisende Verbindungen (3) erhalten werden,
und in einem zweiten Schritt,
die tertiären Stickstoffatome aus den im ersten Schritt erhaltenen Verbindungen (3) mit Alkylierungsmitteln (4) teilweise oder vollständig quaterniert werden.

Vorzugsweise ist R ein Alkylrest, bevorzugt ein linearer Alkylrest, mit mindestens 6 bis 28 C-Atomen, bevorzugt 10 bis 26 C-Atomen und besonders bevorzugt 12 bis 20 C-Atomen.

Bevorzugt ist R¹ ist ein Alkylrest mit 1 bis 18 C-Atomen.

Vorzugsweise sind R³ und R⁴ Alkylreste oder Cycloalkylreste. Bevorzugt ist mindestens ein Rest aus der Gruppe R³, R⁴, R⁵ ein Methyl- oder Ethylrest.

Beispiele für Reste R sind Alkylreste, wie Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl- und Methylcyclöhexylreste; Alkenylreste, wie der Vinyl-, 5-Hexenyl-, Cyclohexenyl-, 1-Propenyl-, Allyl-, 3-Butenyl- und 4-Pentenylrest; und Alkinylreste, wie der Ethinyl-, Propargyl- und 1-Propinylrest.

Beispiele für Kohlenwasserstoffreste R¹ sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neoPentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl- und Methylcyclohexylreste; Alkenylreste, wie der Vinyl-, 5-Hexenyl-, Cyclohexenyl-, 1-Propenyl-, Allyl-, 3-Butenyl- und 4-Pentenylrest; Alkinylreste, wie der Ethinyl-, Propargyl- und 1-Propinylrest; Arylreste, wie der Phenyl-, Naphthyl-, Anthryl- und Phenanthrylrest; Alkarylreste, wie o-, m-, p-Tolylreste, Xylylreste und Ethylphenylreste; und Aralkylreste, wie der Benzylrest, der α- und der β-Phenylethylrest.

Beispiele für Kohlenwasserstoffreste R¹ gelten im vollen Umfang für Kohlenwasserstoffreste R³ und R⁴.

Beispiele für zweiwertige Kohlenwasserstoffreste R² sind Alkylenreste der Formel -CH₂-, -CH₂CH₂-, -CH₂CH₂CH₂-, -CH₂CH₂CH₂CH₂-, -CH₂CH(CH₃) -, und Beispiele für zweiwertige Kohlenwasserstoffreste R² , die ein oder mehrere separate Sauerstoffatome enthalten können, sind solche der Formel -CH₂CH₂OCH₂CH₂-, -CH₂CH(CH₃)OCH₂CH(CH₃)-, CH₂CH(CH₃)OCH₂CH₂-, -CH₂CH₂OCH₂CH₂OCH₂CH₂-.

Beispiele für Alkyl-Keten-Dimere (1) sind solche, die sich von C₈- bis C₃₀-Carbonsäuren ableiten. Die Kohlenwasserstoffreste R können linear, verzweigt oder cyclisch sein, lineare Reste sind bevorzugt. Sie können gesättigt, sowie einfach oder mehrfach ungesättigt sein, wobei gesättigt lineare Kohlenwasserstoffreste bevorzugt sind. Bevorzugte Alkyl-Keten-Dimere (1) leiten sich von C₁₂- bis C₂₈-Carbonsäuren ab, wobei solche auf Basis von C₁₄- bis C₂₂-Carbonsäuren besonders bevorzugt sind. Die Herstellung von Alkyl-Keten-Dimere (1) aus Carbonsäuren ist in US 5,028,236 beschrieben. Allgemein gilt, dass die Anzahl der C-Atome im Rest R der um 2 verminderten Anzahl der C-Atome der den Alkyl-Keten-Dimeren (1) zugrunde liegenden Carbonsäuren entspricht. Anstatt eines Alkyl-Keten-Dimeren mit definierter Kettenlänge R können auch Gemische aus verschiedenen Alkyl-Keten-Dimere (1) mit jeweils verschieden langen R eingesetzt werden, wie sie z. B. aus Carbonsäuregemischen erhalten werden, die aus natürlichen Quellen stammen. Geeignete Fettalkyldiketene sind in EP 1 259 672 B1, Seite 2, Zeilen 52-57 beschrieben.

Die im ersten Schritt des erfindungsgemäßen Verfahrens eingesetzten Aminoverbindungen (2) enthalten mindestens eine tertiäre Aminogruppe und zusätzlich noch mindestens eine protische Gruppe ausgewählt aus der Gruppe der Formeln -OH, -NH₂, -NHR und -NH-. Diese protische Gruppe dient dazu, in einer ersten Stufe mit dem Alkyl-Keten-Dimer (1) zu reagieren und damit mindestens eine tertiäre Aminogruppe an das Alkyl-Keten-Dimer zu binden. Die im ersten Schritt des erfindungsgemäßen Verfahrens erhaltene Verbindung (3) enthält also mindestens eine tertiäre Aminogruppe. Im zweiten Schritt des erfindungsgemäßen Verfahrens werden die so gebundenen tertiären Aminogruppen mit Alkylierungsmitteln (4) teilweise oder vollständig quaterniert.

Bevorzugt werden im ersten Verfahrensschritt als Aminoverbindungen (2) solche der allgemeinen Formel

[R³R^{4'}N-R²-] ₐY-H (IV)

eingesetzt, wobei
- R^{4'} gleich R³: ist oder einen Rest der Formel -R¹-Y-H oder -R¹-NR³₂ bedeutet, und
- a, R¹, R², R³ und Y: die oben dafür angegebene Bedeutung haben.

Beispiele für tertiäre Aminoverbindungen (2), die noch mindestens eine weitere protische Stickstofffunktion tragen sind:
3-Dimethylaminopropylamin,
3-Dimethylaminopropyl-butylamin,
Bis-(3-dimethylaminopropyl)amin,
Bis-(3-aminopropyl)-methylamin,
3-Diethylaminopropyl-1-methylamin,
2-Diethylaminoethylamin,
3-Aminopropyl-3-dimethylaminopropylamin.

Beispiele für tertiäre Aminoverbindungen (2), die noch mindestens eine weitere OH-Gruppe tragen sind:
2-Diethylaminoethanol,
2-Dimethylaminoethanol,
3-Dimethylaminopropanol,
2-Dimethylamino-1-methylethanol,
Bis-(hydroxyethyl)-methylamin,
2-Cyclohexylaminoethanol,
2-Morpholinoethanol,
2-((2-Dimethylaminoethoxy)ethanol,
N,N,N-Trimethyl-N-hydroxyethyl-bisaminoethylether,
Bis-(3-dimethylaminopropyl)-hydroxyethylamin,
3-Dimethylaminopropyl-bis-(2-hydroxyethyl)amin
3-Dimethylaminopropyl-2-hydroxyethylamin,
Tris-(2-hydroxyethyl)amin.

Weitere Beispiele für tertiäre Aminoverbindungen (2) sind die genannten Aminoverbindungen in ihrer ethoxylierten oder propoxylierten Form.

Geeignet als im zweiten Verfahrensschritt eingesetzte Alkylierungsmittel (4) sind alle Verbindungen, die quaternierend auf tertiäre Aminogruppen wirken.

Vorzugsweise werden als Alkylierungsmittel (4) solche der Formel

R⁵-X (V),

eingesetzt, wobei
- R⁵: die oben dafür angegebene Bedeutung hat, und
- X: ein Rest, der bei der Alkylierung der tertiären Stickstoffatome in den Verbindungen (3) ein Gegenion X⁻ zu der positiven Ladung am quartären Stickstoffatom bildet.

Bevorzugt ist R⁵ ein linearer, verzweigter oder cyclischer Alkylrest mit 1 bis 6 Kohlenstoffatomen.
Beispiele für Reste R⁵ sind der Methyl-, Ethyl-, Propyl-, Isopropyl-, Butyl-, Isobutyl und der Cyclohexylrest.

Beispiele für geeignete Alkylierungsmittel (4) sind Dialkylsulfate, wie Dimethylsulfat und Diethylsulfat, Sulfonsäureester, wie p-Toluolsulfonsäuremethylester , p-Toluolsulfonsäureethylester , p-Toluolsulfonsäurepropylester, sowie auch Benzylverbindungen, wie Benzylchlorid , Benzylbromid und Benzyljodid. Besonders bevorzugt sind Dimethylsulfat, Diethylsulfat und p-Toluolsulfonsäuremethylester.

Beispiele für Gegenionen X⁻ zur positiven Ladung am quartären Stickstoffatom sind
CH₃SO₄⁻
CH₃CH₂SO₄⁻
C₆H₅SO₃⁻
p-CH₃(C₆H₄)SO₃⁻
CH₃SO₃⁻
C₄H₉SO₃⁻
C₈H₁₇SO₃⁻
Cl⁻, Br⁻ und J⁻.

Das erfindungsgemäße zweistufige Verfahren kann in zwei getrennten Syntheseschritten erfolgen, wie auch bevorzugt in einem Tandemverfahren in einer Reaktionsanlage. Da die Alkyl-Keten-Dimere (1) bei 25°C meist fest sind, erfolgt der erste Verfahrensschritt vorzugsweise bei erhöhter Temperatur im Bereich von 40 bis 140°C, bevorzugt bei 50 bis 120°C.
Bevorzugt findet der zweite Verfahrensschritt bei 60 bis 140°C statt. Falls erwünscht, lässt sich die gesamte Synthese ohne zwischenzeitliches Abkühlen vorteilhaft in einem Zug durchfahren.
Das erfindungsgemäße Verfahren wird vorzugsweise beim Druck der umgebenden Atmosphäre, also bei etwa 1020 hPa durchgeführt, kann aber auch bei höheren oder niedrigeren Drücken durchgeführt werden.

In ersten Schritt des erfindungsgemäßen Verfahrens wird Alkyl-Keten-Dimer (1) in Mengen von vorzugsweise 0,8 bis 1,2 Mol Diketen, bevorzugt 0,9 bis 1,1 Mol Diketen, je Mol protische Gruppe in der Aminoverbindung (2) eingesetzt. Alkyl-Keten-Dimere (1) werden besonders bevorzugt equimolar zu den in den Aminoverbindungen (2) enthaltenen protischen Gruppen eingesetzt. Zu berücksichtigen ist dabei, dass (1) selten reines Alkyl-Keten-Dimer ist, und meist mit einer Reinheit von 85 bis 95% im Handel erhältlich ist.

Im zweiten Schritt des erfindungsgemäßen Verfahrens wird Alkylierungsmittel (4) vorzugsweise in Mengen von 0,8 bis 1,0 Mol, bevorzugt 0,9 bis 1,0 Mol, je Mol tertiäres Stickstoffatom in der Verbindung (3) eingesetzt. Zu berücksichtigen ist auch hier die Reinheit des Alkylierungsmittels, so wie auch die der übrigen Komponenten. Sollten diese Verunreinigungen enthalten, die mit dem Alkylierungsmittel ebenfalls reagieren, können zur vollständigen Quaternierung der tert. Stickstoffatome auch mehr als die bevorzugten 1,0 Mol Alkylierungsmittel je Mol tert. Stickstoffatom erforderlich sein.

Das erfindungsgemäße Verfahren hat gegenüber dem Verfahren zur Herstellung der bekannten Esterquats, wie sie z. B. in US 2009/0247629 A1 beschrieben sind, den Vorteil, dass die Amidierung im ersten Verfahrensschritt in weniger als 30 Minuten bei 60°C verläuft gegenüber US 2009/0247629 A1, in der die Veresterung, die noch dazu immer unvollständig ist, bei 200°C über viele Stunden (3-8 Stunden) erfolgt und die anschließende Umsetzung des Esteramins zur Esterquatverbindung weitere 6-9 Stunden bei 90°C benötigt.

Ein weiterer Vorteil ist, dass erfindungsgemäß eine nahezu quantitative Quaternierung erzielt wird gegenüber einem 60-90% Quaternierungsgrad bei der Herstellung der Esterquats gemäß Stand der Technik.
Dies bedeutet, dass keine Überschüsse an Alkylierungsmittel (4) eingesetzt werden müssen, um eine nahezu quantitative Quaternierung zu erreichen, und daher im Endprodukt keine toxischen Alkylierungsmittel (4) enthalten sind. Auch bei einer absichtlich knappen Unterdosierung des Alkylierungsmittels (4) werden immer noch sehr hohe Ausbeuten erzielt.
Damit ist eine sehr hohe Aktivkonzentration realisierbar und damit entsprechend wenig Belastung der Abwässer durch Nebenprodukte.

Ein weiterer Vorteil ist, das die erfindungsgemäßen β-Ketocarbonylquatverbindungen biologisch abbaubar sind.

Die erfindungsgemäßen β-Ketocarbonylquatverbindungen weisen eine überraschend hohe Hydrophilie auf im Vergleich zu den bekannten Esterquats, die hydrophob sind. Überraschend ist die ausgezeichnete Hydrophilie auch deswegen, weil Alkyl-Keten-Dimer-Dispersionen seit vielen Jahren zur kontrollierten Hydrophobierung von Papier weltweit eingesetzt werden (Papierleimung), und vergleichbare Anwendungen aus CH 388246 und US 5,028,236 bekannt sind.

In ihrer bevorzugten Amidform sind die erfindungsgemäßen β-Ketocarbonylquatverbindungen auch erheblich hydrolysestabiler als die handelsüblichen Esterquats, die daher nur in einem engen pH-Bereich gehandhabt und angewendet werden können.

Durch das erfindungsgemäße Verfahren können auch bakterizide β-Ketocarbonylquatverbindungen mit einer längeren Alkylkette R hergestellt werden.

Die exzellent abgestufte Reaktivität des Alkyl-Keten-Dimers lässt es auch zu, durch den Einsatz von Aminoverbindung (2) mit OH-Gruppen, wie Dimethylaminoethanol, entsprechende Alkyl-Keten-Dimer-Esterquats herzustellen, falls Estergruppen zwischen dem Hydrophob- und dem Hydrophilteil gewünscht sind.

Die β-Ketoamidstruktur in den erfindungsgemäßen β-Ketocarbonylquatverbindungen hat den Vorteil, dass sie zur Komplexierung von Metall-ionen dienen kann. Hierdurch sind weitere Fixierungsmöglichkeiten auf Oberflächen gegeben.

### Beispiel 1:

119 g Alkyl-Keten-Dimer mit einem Diketenequivalentgewicht von 570 g/Mol (Alkylrest R ca. 16 C-Atome, käuflich erwerblich als "AKD" bei Fa. Trigon Chemie GmbH) werden vorsichtig ohne Überhitzung aufgeschmolzen. Bei 63°C werden 21,4 g 3-Dimethylaminopropylamin langsam unter Kühlung und gutem Rühren zudosiert, so dass eine Innentemperatur von 75°C nicht überschritten wird. Wenige Minuten nach Beendigung der Zudosierung ist die Amidierung abgeschlossen. Es wird ein Amidoamin mit einer Aminzahl von 1,48 (theoretisch 1,49) erhalten, was eine praktisch 100 %-ige Ausbeute bedeutet.
Das erhaltene Amidoamin wird auf 100°C erwärmt und es werden insgesamt 37,2 g p-Toluolsulfonsäuremethylester (0,95 Mol je Mol tertiäre Aminogruppe in dem Amidoamin) über einen Zeitraum von 30 Minuten zugegeben. Man lässt weitere 2 Stunden bei 100°C nachreagieren und erhält 177,6 g eines Dialkylacetoacetamidoquats, das sich beim Abkühlen verfestigt. Das Produkt hat eine Aminzahl von 0,07, was einer Quaternierung von ca. 94 % entspricht. Dieser Wert stimmt gut mit der beabsichtigten 5 %-igen Unterdosierung des p-Toluolsulfonsäuremethylesters (MeOTs) überein.

### Beispiel 2:

Zu 119 g des in Beispiel 1 beschriebenen Alkyl-Keten-Dimer (570g/Mol Diketen) werden bei 61°C langsam insgesamt 39,2 g Bis-(3-dimethylamino-propyl)amin (käuflich erwerblich unter dem Handelsnamen "JEFFCAT Z 130" bei der Fa. Huntsman) dosiert. Die exotherme Reaktion der Amidierung ist kurz nach Beendigung der Dosierende abgeschlossen. Danach werden zu dem erhaltenen Amidoamin bei 100°C 74,5 g p-Toluolsulfonsäuremethylester (0,95 Mol je Mol tertiäre Aminogruppe in dem Amidoamin) portionsweise dosiert, wobei das Reaktionsgemisch 118°C erreicht. Bei 100°C lässt man 2 Stunden lang ausreagieren. Gemäß einer Aminzahl von 0,11 sind ca. 94 % der tertiären Aminogruppen quaterniert.

### Beispiel 3:

Die Arbeitsweise von Beispiel 1 wird wiederholt mit der Abänderung, dass die Menge des Alkylierungsmittels aber auf 100% (39,0 g) erhöht wird und damit gleichmolar zur Menge des tertiären Amins eingesetzt wird. Es werden 179,4 g des gleichen Dialkylacetoacetamidoquats wie in Beispiel 1 erhalten, aber mit einer Aminzahl von nur 0,02, was einem Quaternierungsgrad von 98 % entspricht.

### Beispiel 4:

Bei 65°C werden 119 g des in Beispiel 1 beschriebenen aufgeschmolzenen Alkyl-Keten-Dimers (570g / Mol Diketen) mit 18,6 g 2-Dimethylaminoethanol umgesetzt, das über einen Zeitraum von 12 Minuten zudosiert wird. Durch die sofort einsetzende exotherme Reaktion erwärmt sich das Gemisch trotz Kühlung um 6°C. Nach einer Stunde bei 100°C dosiert man in den Alkyl-Keten-Dimer-Aminoester 37 g p-Toluolsulfonsäuremethyl-ester und lässt weitere 2 Stunden nachreagieren, was mit einem deutlichen Viskositätsanstieg einher geht. Es werden 174,6 g eines beim Abkühlen fest werdenden Esterquats erhalten mit einer Aminzahl von 0,07 und vollständig umgesetzten Alkylierungsmittel (p-Toluolsulfonsäuremethylester). Dies entspricht einer Quaternierung von rund 94 %, entsprechend der 5%-igen Unterdosierung des p-Toluolsulfonsäuremethylester (MeOTs).

Wie die Beispiele zeigen, können mit dem erfindungsgemäßen Verfahren sowohl Amidoquats wie auch Esterquats schnell und in sehr hohen Ausbeuten hergestellt werden. Auch der Quaternierungsgrad ist gut steuerbar und kann bis zu quantitativen Umsätzen gefahren werden.

## Patentansprüche

1. β-Ketocarbonylquatverbindungen, die eine oder mehrere β-Ketocarbonylgruppen der allgemeinen Formel
R-CH₂-(C=O)-CHR-(C=O)- (I)
und
eine oder mehrere quartäre Ammoniumgruppen enthalten, wobei
R gleich oder verschieden sein kann und einen aliphatischen Kohlenwasserstoffrest mit 6 bis 28 C-Atomen, vorzugsweise 10 bis 26 C-Atomen, bevorzugt 12 bis 20 C-Atomen, bedeutet,
mit der Maßgabe, dass die β-Ketocarbonylgruppe der Formel (I) an einen Rest Y gebunden ist, wobei
Y einen zweiwertigen Rest der Formel -O-, -NH-, -NR¹-, bevorzugt -NH-, -NR¹-, oder einen dreiwertigen Rest der Formel =N- bedeutet, und
R¹ einen einwertigen Kohlenwasserstoffrest mit 1 bis 30 C-Atomen, vorzugsweise 1 bis 18 C-Atomen, bedeutet.

2. β-Ketocarbonylquatverbindungen nach Anspruch 1, **dadurch gekennzeichnet, dass** es solche der allgemeinen Formel
[R³R⁴R⁵N⁽⁺⁾-R²-] ₐY-Z X⁽⁻⁾ (II),
sind, wobei
a 1 oder 2 ist, mit der Maßgabe, dass wenn a=1, Y ein zweiwertiger Rest ist und wenn a=2, Y ein dreiwertiger Rest ist,
Y einen zweiwertigen Rest der Formel -O-, -NH-, -NR¹-, oder einen dreiwertigen Rest der Formel =N- bedeutet,
X⁽⁻⁾ Gegenion zu der positiven Ladung am quartären Stickstoffatoms ist,
Z eine β-Ketocarbonylgruppe der Formel R-CH₂-(C=O)-CHR-(C=O)- (I) bedeutet,
R gleich oder verschieden sein kann und einen aliphatischen Kohlenwasserstoffrest mit 6 bis 28 C-Atomen, vorzugsweise 10 bis 26 C-Atomen, bevorzugt 12 bis 20 C-Atomen, bedeutet,
R¹ einen einwertigen Kohlenwasserstoffrest mit 1 bis 30 C-Atomen, vorzugsweise 1 bis 18 C-Atomen, bedeutet,
R² einen zweiwertigen C₁-C₁₈-Kohlenwasserstoffrest, der ein oder mehrere separate Sauerstoffatome enthalten kann, bedeutet,
R³ gleich oder verschieden ist und einen einwertigen Kohlenwasserstoffrest mit 1 bis 30 C-Atomen, vorzugsweise 1 bis 18 C-Atomen, bedeutet,
R⁴ gleich R³ ist oder einen Rest der Formel -R¹-Y-Z oder -R¹-N⁽⁺⁾R³₂R⁵ X⁽⁻⁾ bedeutet, oder R³ und R⁴ zusammen oder zwei Reste R³ zusammen einen zweiwertigen C₃-C₁₂-Kohlenwasserstoffrest, der gegebenenfalls ein O-Atom oder N-Atom enthalten kann, bedeuten und
R⁵ einen einwertigen Kohlenwasserstoffrest mit 1 bis 18 C-Atomen, vorzugsweise 1 bis 12 C-Atomen, bedeutet.

3. β-Ketocarbonylquatverbindungen nach Anspruch 1 oder 2,
**dadurch gekennzeichnet, dass**
Y in Formel (II) -NH-, -NR¹- oder =N- bedeutet,
wobei R¹ einen einwertigen Kohlenwasserstoffrest mit 1 bis 30 C-Atomen, vorzugsweise 1 bis 18 C-Atomen, bedeutet.

4. Verfahren zur Herstellung der β-Ketocarbonylquatverbindungen,
indem in einem ersten Schritt
Alkyl-Keten-Dimere (1) der allgemeinen Formel 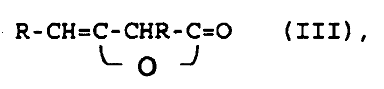 wobei R gleich oder verschieden sein kann und einen aliphatischen Kohlenwasserstoffrest mit 6 bis 28 C-Atomen, vorzugsweise 10 bis 26 C-Atomen, bevorzugt 12 bis 20 C-Atomen, bedeutet,
mit Aminoverbindungen (2), die mindestens eine tertiäre Aminogruppe und mindestens eine protische Gruppe ausgewählt aus der Gruppe der Formeln -OH , -NH₂ , -NHR¹ und -NHenthalten, wobei R¹ einen einwertigen Kohlenwasserstoffrest mit 1 bis 30 C-Atomen, vorzugsweise 1 bis 18 C-Atomen, bedeutet,
umgesetzt werden, wobei tertiäre Aminogruppen aufweisende Verbindungen (3) erhalten werden,
und in einem zweiten Schritt,
die tertiären Stickstoffatome aus den im ersten Schritt erhaltenen Verbindungen (3) mit Alkylierungsmitteln (4) teilweise oder vollständig quaterniert werden.

5. Verfahren nach Anspruch 4, **dadurch gekennzeichnet, dass** im ersten Schritt des Verfahrens als Aminoverbindungen (2) solche der allgemeinen Formel
[R³R^{4'}N-R²-] ₐY-H (IV)
eingesetzt, wobei
R⁴' gleich R³ ist oder einen Rest der Formel -R¹-Y-H oder -R¹-NR³₂ bedeutet, und
a, R¹, R², R³ und Y die im Anspruch 2 dafür angegebene
Bedeutung haben.

6. Verfahren nach Anspruch 4 oder 5, **dadurch gekennzeichnet, dass** im zweiten Schritt des Verfahrens als Alkylierungsmittel (4) solche der Formel
R⁵-X (V),
eingesetzt werden, wobei
R⁵ einen einwertigen Kohlenwasserstoffrest mit 1 bis 18 C-Atomen, vorzugsweise 1 bis 12 C-Atomen, bedeutet, und
X ein Rest ist, der bei der Alkylierung der tertiären Stickstoffatome in den Verbindungen (3) ein Gegenion X⁻ zu der positiven Ladung am quartären Stickstoffatom bildet.

7. Verfahren nach Anspruch 4, 5 oder 6, **dadurch gekennzeichnet, dass** im zweiten Schritt des Verfahrens als Alkylierungsmittel (4) Dialkylsulfate und Sulfonsäureester eingesetzt werden.

## Claims

1. β-Ketocarbonylquats containing one or more β-ketocarbonyl groups of general formula
R-CH₂-(C=O)-CHR-(C=O)- (I)
and
one or more quaternary ammonium groups, wherein
R is an aliphatic hydrocarbon radical of 6 to 28 carbon atoms, preferably 10 to 26 carbon atoms and more preferably 12 to 20 carbon atoms and may be the same or different in each occurrence,
with the proviso that the β-ketocarbonyl group of formula (I) is bonded to a radical Y, wherein
Y is a divalent radical of the formula -O-, -NH-, -NR¹-, preferably -NH-, -NR¹-, or a trivalent radical of the formula =N-, and
R¹ is a monovalent hydrocarbon radical of 1 to 30 carbon atoms, preferably 1 to 18 carbon atoms.

2. β-Ketocarbonylquats according to Claim 1, **characterized in that** they have the general formula
[R³R⁴R⁵N⁽⁺⁾-R²-]ₐY-Z X⁽⁻⁾ (II)
wherein
a is 1 or 2, with the proviso that when a=1, Y is a divalent radical and when a=2, Y is a trivalent radical,
Y is a divalent radical of formula -O-, -NH-, -NR¹-, or a trivalent radical of formula =N-,
X⁽⁻⁾ is a counter-ion to the positive charge on the quaternary nitrogen atom,
Z is a β-ketocarbonyl group of the formula R-CH₂-(C=O)-CHR-(C=O)- (I)
R is an aliphatic hydrocarbon radical of 6 to 28 carbon atoms, preferably 10 to 26 carbon atoms and more preferably 12 to 20 carbon atoms and may be the same or different in each occurrence,
R¹ is a monovalent hydrocarbon radical of 1 to 30 carbon atoms, preferably 1 to 18 carbon atoms,
R² is a divalent C₁-C₁₈ hydrocarbon radical which may contain one or more separate oxygen atoms,
R³ is a monovalent hydrocarbon radical of 1 to 30 carbon atoms, preferably 1 to 18 carbon atoms, and is the same or different in each occurrence,
R⁴ is R³ or a radical of the formula -R¹-Y-Z or -R¹-N⁽⁺⁾R³₂R⁵ X⁽⁻⁾, or R³ and R⁴ together or two R³ radicals together are a divalent C₃-C₁₂ hydrocarbon radical which may optionally contain an oxygen atom or a nitrogen atom, and
R⁵ is a monovalent hydrocarbon radical of 1 to 18 carbon atoms, preferably 1 to 12 carbon atoms.

3. β-Ketocarbonylquats according to Claim 1 or 2,
**characterized in that**
Y in formula (II) is -NH-, -NR¹- or =N-,
wherein R¹ is a monovalent hydrocarbon radical of 1 to 30 carbon atoms, preferably 1 to 18 carbon atoms.

4. Process for preparing the β-ketocarbonylquats, which process comprises a first step of reacting alkyl-ketene dimmers (1) of general formula 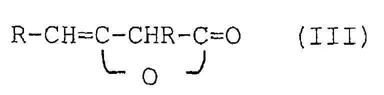 wherein R is an aliphatic hydrocarbon radical of 6 to 28 carbon atoms, preferably 10 to 26 carbon atoms and more preferably 12 to 20 carbon atoms and may be the same or different in each occurrence,
with amino compounds (2) which contain at least one tertiary amino group and at least one protic group selected from the group consisting of the formulae -OH, -NH₂, -NHR¹ and -NH-, wherein R¹ is a monovalent hydrocarbon radical of 1 to 30 carbon atoms, preferably 1 to 18 carbon atoms,
to obtain compounds (3) comprising tertiary amino groups,
and a second step of
partly or wholly quaternizing the tertiary nitrogen atoms from said compounds (3) obtained in the first step, with alkylating agents (4).

5. Process according to Claim 4, **characterized in that** amino compounds (2) used in the first step of the process have the general formula
[R³R^{4'}N-R²-]ₐY-H (IV)
wherein
R^{4'} is R³ or a radical of the formula -R¹-Y-H or -R¹-NR³₂, and
a, R¹, R², R³ and Y are each as defined in Claim 2.

6. Process according to Claim 4 or 5, **characterized in that** alkylating agents (4) used in the second step of the process have the formula
R⁵-X (V)
wherein
R⁵ is a monovalent hydrocarbon radical of 1 to 18 carbon atoms, preferably of 1 to 12 carbon atoms, and
X is a radical which in the step of alkylating the tertiary nitrogen atoms in said compounds (3) forms a counter-ion X⁻ to the positive charge on the quaternary nitrogen atom.

7. Process according to Claim 4, 5 or 6, **characterized in that** alkylating agents (4) used in the second step of the process are dialkyl sulfates and sulfonic esters.

## Revendications

1. Composés quaternaires de β-cétocarbonyle, qui contiennent un ou plusieurs groupes β-cétocarbonyle de formule générale
R-CH₂-(C=O)-CHR-(C=O)- (I)
et
un ou plusieurs groupes ammonium quaternaires,
les R pouvant être identiques ou différents, et signifiant un radical hydrocarboné aliphatique de 6 à 28 atomes C, avantageusement de 10 à 26 atomes C, de préférence de 12 à 20 atomes C,
à condition que le groupe β-cétocarbonyle de formule (I) soit relié à un radical Y,
Y signifiant un radical bivalent de formule -O-,-NH-, -NR¹-, de préférence -NH-, -NR¹-, ou un radical trivalent de formule =N-, et
R¹ signifiant un radical hydrocarboné monovalent de 1 à 30 atomes C, avantageusement de 1 à 18 atomes C.

2. Composés quaternaires de β-cétocarbonyle selon la revendication 1, **caractérisés en ce qu'**il s'agit de composés de formule générale
[R³R⁴R⁵N⁽⁺⁾-R²-]ₐY-Z X⁽⁻⁾ (II)
dans laquelle
a représente 1 ou 2, à condition que
lorsque a = 1, Y représente un radical bivalent et
lorsque a = 2, Y représente un radical trivalent,
Y signifie un radical bivalent de formule -O-, -NH-, -NR¹- ou un radical trivalent de formule =N-,
X⁽⁻⁾ représente un contre-ion de la charge positive sur l'atome d'azote quaternaire,
Z signifie un groupe β-cétocarbonyle de formule
R-CH₂-(C=O) -CHR- (C=O) - (I)
les R peuvent être identiques ou différents, et signifient un radical hydrocarboné aliphatique de 6 à 28 atomes C, avantageusement de 10 à 26 atomes C, de préférence de 12 à 20 atomes C,
R¹ signifie un radical hydrocarboné monovalent de 1 à 30 atomes C, avantageusement de 1 à 18 atomes C,
R² signifie un radical hydrocarboné en C₁-C₁₈ bivalent, qui peut contenir un ou plusieurs atomes d'oxygène séparés,
les R³ sont identiques ou différents et signifient un radical hydrocarboné monovalent de 1 à 30 atomes C, avantageusement de 1 à 18 atomes C,
R⁴ est identique à R³ ou signifie un radical de formule -R¹-Y-Z ou -R¹-N⁽⁺⁾R³₂R⁵ X⁽⁻⁾,
ou R³ et R⁴ signifient ensemble ou deux radicaux R³ signifient ensemble un radical hydrocarboné en C₃-C₁₂ bivalent, qui peut éventuellement contenir un atome O ou un atome N, et
R⁵ signifie un radical hydrocarboné monovalent de 1 à 18 atomes C, avantageusement de 1 à 12 atomes C.

3. Composés quaternaires de β-cétocarbonyle selon la revendication 1 ou 2, **caractérisés en ce que**
Y dans la formule (II) signifie -NH-, -NR¹- ou =N-,
R¹ signifiant un radical hydrocarboné monovalent de 1 à 30 atomes C, avantageusement de 1 à 18 atomes C.

4. Procédé de fabrication des composés quaternaires de β-cétocarbonyle,
selon lequel, lors d'une première étape,
des dimères d'alkyl-cétène (1) de formule générale 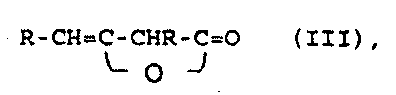 dans laquelle les R peuvent être identiques ou différents, et signifient un radical hydrocarboné aliphatique de 6 à 28 atomes C, avantageusement de 10 à 26 atomes C, de préférence de 12 à 20 atomes C,
sont mis en réaction avec des composés amino (2), qui contiennent au moins un groupe amino tertiaire et au moins un groupe protique choisi dans le groupe des formules -OH, -NH₂, -NHR¹ et -NH-, R¹ signifiant un radical hydrocarboné monovalent de 1 à 30 atomes C, avantageusement de 1 à 18 atomes C,
des composés (3) comprenant des groupes amino tertiaires étant obtenus,
et, lors d'une seconde étape,
les atomes d'azote tertiaires des composés (3) obtenus lors de la première étape sont quaternisés en partie ou en totalité avec des agents d'alkylation (4).

5. Procédé selon la revendication 4, **caractérisé en ce que**, lors de la première étape du procédé, des composés de formule générale
[R³R⁴N-R²-]ₐY-H (IV)
sont utilisés en tant que composés amino (2),
R^{4'} étant identique à R³ ou signifiant un radical de formule -R¹-Y-H ou -R¹-NR³₂, et
a, R¹, R², R³ et Y ayant la signification indiquée dans la revendication 2.

6. Procédé selon la revendication 4 ou 5, **caractérisé en ce que**, lors de la seconde étape du procédé, des composés de formule
R⁵-X (V)
sont utilisés en tant qu'agents d'alkylation (4),
R⁵ signifiant un radical hydrocarboné monovalent de 1 à 18 atomes C, avantageusement de 1 à 12 atomes C, et
X représentant un radical qui forme un contre-ion X⁻ de la charge positive sur l'atome d'azote quaternaire lors de l'alkylation des atomes d'azote tertiaires dans les composés (3).

7. Procédé selon la revendication 4, 5 ou 6, **caractérisé en ce que**, lors de la seconde étape du procédé, des sulfates de dialkyle et des esters de l'acide sulfonique sont utilisés en tant qu'agents d'alkylation (4).
